# EUROPEAN PATENT APPLICATION

(11) **EP 0 712 928 A2**
(43) Date of publication of application: **22.05.1996**
(21) Application number: 95112520.2
(22) Date of filing: 21.06.1988
(51) Int. Cl.: C12N 15/00, C12N 5/00, C12P 21/00

(54) **T cell antigen receptor**

(30) Priority: 23.06.1987 US 65407
(62) Divisional of application: 88305622.8
(71) Applicant: THE BOARD OF TRUSTEES OF THE LELAND STANFORD JUNIOR UNIVERSITY, Stanford California 94305 (US)
(72) Inventor: Davis, Mark, Mountain View, California 94041 (US); Chien, Yueh-hsiu, Mountain View, California 94041 (US)
(74) Representative: Silveston, Judith

(57) **Abstract**

DNA sequences encoding T-cell specific peptides are disclosed. The peptides are normally associated with an immature differentiation stage of T-cells. The DNA sequences encoding the peptides may be used in constructs for production of the peptides, by themselves or in combination with other peptides, particularly to provide T-cell receptors.

## Description

The identification of sequences specifically expressed in T-cells and the use of the sequence in the expression of a putative receptor.

The thymus is the major differentiative organ for T-lymphocytes in higher vertebrates. The regulation of expression and assembly of the T-cell antigen receptor (TCR) genes must relate to the many selective and maturational events which take place in the thymus as T-cell precursors progress through various stages of differentiation. Two distinct types of T-cell antigen receptors (TCR) have thus far been identified: the α:β heterodimer found on functional helper and cytotoxic T-cells, and the more recently described γ:δ heterodimer. Both heterodimers are independently expressed on the cell surface of distinct populations of T-cells in association with the monomorphic CD3 polypeptides.

Much has been reported concerning the α:β heterodimer. Sequences have been isolated encoding each of the subunits of the α:β heterodimer and been used for expression of the α:β TCR. Furthermore, quite early in the investigations concerning the TCRs, a sequence was discovered, which was reported to be the γ subunit of a putative TCR different from the α:β receptor. The γ:δ receptor appears to be different in a number of significant ways from the α:β receptor. The γ:δ receptor does not seem to show MHC-restricted cytotoxicity. The γ:δ receptor is normally detected prior to the appearance of the α:β receptor during T-cell ontogeny. Because of the substantial differences between the γ:δ receptor and the α:β, there is substantial interest in being able to produce the γ:δ heterodimer as a receptor, which may find use in diagnosis and therapy, as a source of binding proteins, as an agonist or antagonist of T-cell binding, and in an understanding of the mechanisms involved with T-cell etiology and function.

### Relevant Literature

The γ:δ heterodimer is described by Brenner et al., Nature (1986) 322:145-149; Lew et al., Science (1986) 234:1401-1405; Pardoll et al., Nature (1987) 326:79-81; Bluestone et al., Nature (1987) 326:82-84; Borst et al., Nature (1987) 325:683-688; Moingeon et al., Nature (1987) 325:723-726. The γ gene is described by Saito et al., Nature (1984) 309:757-762; Sim et al., Nature (1984) 312:771-775.

### SUMMARY OF THE INVENTION

Novel DNA sequences and constructs are provided for the expression of a surface protein expressed early in the maturation of T-cells which may serve as the δ subunit of the γ:δ heterodimer receptor. The constructs may be used to simultaneously produce the γ and δ subunits in an appropriate host for expression of the γ:δ T-cell receptor or functional fragments thereof. The DNA sequence may also be used as a probe.

### DESCRIPTION OF THE SPECIFIC EMBODIMENTS

Novel DNA sequences, constructs, and expression systems are provided associated with a sequence and encoding a peptide associated with an early stage of T-cell maturation. The sequence is located 5' of the J_{α}C_{α} coding regions, which shows systematic rearrangement in early thymocytes, employing V gene segments of the TCR α family, before any C_{α} message or protein is detected. RNA from this locus is expressed at a high level in early thymocytes and adult CD4⁻, CD8⁻ cells but not in mature T-cell populations.

The sequence appears to be the leader when V_{α} -> J_{α} rearrangement occurs. Rearrangement at this locus appears to occur at least as early as day 14 of fetal thymocytes. It therefore appears to be a rearrangement at the same time or perhaps just before TCR β and γ genes, see Haars et al., J. Exp. Med. (1986) 164:1-24 and Born et al., Science (1986) 234:479-482. The rearranged bands become more intense and complex on day 18 and day 19 of thymocyte DNA and diminishing more with mature T-cells.

The gene may be divided into three parts, V, J, and C where the V region may be analogized to the variable region of immunoglobulins and the α subunit of the α:β TCR, the J region to the joining region and the C region to the constant region. The V region has substantial homology with the V_{α} region and similarly the J region has similarity to the J_{α} region.

The genomic sequence includes a conserved heptamer (AGCTGTG) and nonamer sequence (GGTTTTTGG) separated by 13 nucleotides and located 5' of the unrearranged J sequence. At the 3' boundary there is the conserved RNA splicing signal sequence (GTAAGT). A D region is also present separating the V and J regions to provide an exon excompassing the VDJ coding regions.

A C region is observed which exists as a single copy gene. It Is located about 13 kb 3' of the J gene segment and about 70 kb 5' of C_{α}. The coding sequence is estimated to code for a protein of which the amino acid sequence would have a molecular weight of about 30.7 kD. The sequence has two potential N-linked glycosylation sites.

The C region coding sequence shares short regions of homology with each of the previously described TCR or Ig sequences and is between 9-18% homologous at the amino acid sequence level to any one T-cell receptor sequence. It preserves many of the characteristics of T-cell receptor genes with an extra cysteine past the first domain, presumably for heterodimer formation and also including a conserved lysine residue in the transmembrane region, a feature of all of the other T-cell receptor constant regions. The lysine may be important for interactions with CD3 polypeptides. All three of the CD3 peptides characterized to date have a conserved aspartic acid or glutamic acid residue in their apparent transmembrane regions. The C region has a relatively short (14-15 amino acid) hydrophobic C-terminus (counting from the conserved lysine) and has substantial similarity in the way conserved sequences are spaced with those of C_{α}. The subject gene is found in adult dull CD1 cells with hybridomas.

Constructs of interest will usually include at least 8, usually at least 12 nucleotides of the C coding region or non-coding flanking regions, where the C region or fragment thereof may be joined in proper orientation to the J, D and V regions. Constructs may also comprise heterologous DNA, such as a marker for selection, a stable replication system, a synthetic linker or polylinker, a transcriptional initiation region, a transcriptional termination region, or the like. The C region containing fragment will usually be less than about 10 kbp of the original sequence comprising the fragment usually less than about 5 kbp.

The subject nucleic acid sequence may be used in a variety of ways. The sequence may be used as a probe, particularly the C sequence or J sequence to identify mRNA or genomic DNA in various T-cells for determination of the presence of the sequence. The probes would normally be at least about 8 bases, more usually at least about 12 bases, and may be 50 bases or more. Usually, the probes will not exceed about 1 kbp, more usually not exceeding about 0.5 kbp of homologous sequence. The probes will normally be labelled, conveniently labelled with radioisotopes, using various conventional techniques, such as nick translation or TdT extensions with radioactive nucleotides, or the like.

cDNAs which are obtained from mRNA encoding the subject peptide may be used for expression of the subject peptide. The cDNA may be inserted into an appropriate expression vector, by itself or in conjunction with a gene expressing all or a portion of the γ sequence to produce a δ:γ TCR.

A wide variety of expression vectors are available for expression in a wide variety of hosts. Both prokaryotic and eukaryotic hosts are available for expression. Hosts which may be used include E. coli, B. subtilis, B. licheniformis, S. cervisiae, K. lactis, CHO cells, monkey kidney cells, COS cells, etc.

A wide variety of vectors are available for expression, employing a wide variety of transcription initiation regions. Conveniently, the vectors include expression cassettes comprising in the direction of transcription, a transcriptional and translational initiation region, a site for insertion of a gene to be under the regulation of the initiation region, and a translational and transcription termination region. In addition, a marker is usually provided, which allows for selection of expression host cells containing the expression cassette for expression of the subject peptide, the expression cassette being either by itself or in conjunction with an expression cassette for the gene encoding the γ sequence.

Markers providing selection may include sequences which are complementary to a negative background for the gene, so as to impart prototrophy to an auxotrophic host, genes which provide for antibiotic resistance, such as resistance to kanamycin, chloramphenicol, penicillin, G418, tetracycline, gentamicin, etc. Illustrative references include U.S. Patent Nos. 4,615,974; 4,615,980; U.S. 4,624,926 and 4,626,505.

The gene may be manipulated in a variety of ways, by removing all or a portion of the non-coding regions at either or both the 5' or 3'-termini, by in vitro mutagenesis or primer repair, so as to change one or more nucleotides to introduce restriction sites, remove undesirable restriction sites, change one or more codons, by terminal extension, ligation to adapters or linkers, or the like. The sequence may therefore be modified not only by the above techniques, but by restriction, resection, ligation to various functional sequences, or the like. Usually, after each manipulation, the sequence will be cloned and analyzed to ensure that the desired sequence has been obtained. Analysis may include restriction analysis, sequencing, electrophoresis, Southern hybridization, etc.

The expression vector containing the subject polypeptide, either by itself or in conjunction with the sequence encoding a γ polypeptide may be introduced into any of a variety of host cells, where the various regulatory regions and replication systems of the vector are functional. Transformation may be achieved in a variety of ways, depending upon the particular replication system. In some instances, the replication system may not be functional in the host, so that integration is sought, rather than extrachromosomal maintenance. Vectors may be based on plasmids, viruses, phage, chromosomal replication systems, e.g., centromere in conjunction with an ars, or the like. For mammalian expression systems, replication systems from SV40, adenovirus, bovine papilloma virus, or the like may be employed, for example in COS cells with SV40. The transformed cells may be grown in an appropriate medium for expression of the subject peptides. Depending upon the particular host, the peptides may be retained in the cytoplasm or be transported to the membrane of the host to become membrane bound proteins.

The sequence encoded by the subject peptide may be modified by removing one or more regions or parts of one or more regions. Thus, the VD region may isolated or the VDJ region may be isolated, by itself or in conjunction with the variable region of the γ protein. In this manner, a varible region may obtained which lacks all or a portion of the constant region. The variable region may be manipulated by removing the signal leader sequence, or the constant region by removal of the transmembrane sequence, or both, so as to prevent the transport to the membrane and retain the peptide the cytoplasm. Alternatively, the constant region may be joined to a foreign protein to serve as the transport mechanism for transporting the foreign protein to the cytoplasmic membrane. The joining of various fragments is extensively described in the literature and may be achieved in a variety of ways, using linkers or adapters. See, for example, Maniatis et al., Molecular Cloning; A Laboratory Manual, Cold Spring Harbor Laboratory, Cold Spring Harbor, NY, 1982; and U.S. Patent No. 4,617,384.

The peptide products may be used in a variety of ways. The receptors may be used for binding to the complementary ligand. The binding may find use in diagnostic assays, affinity chromatography, or the like. The receptors may be used to compete with T-cells in vitro or in vivo to prevent their binding to the complementary ligand. The subject peptides may be used to transform T-cells to enhance the ability of T-cells to bind to a predetermined ligand. The subject peptides may be used to produce monoclonal or polyclonal antibodies which may then be used to isolate or remove a particular subset of T-cells which share an idiotope or paratope with the subject peptide. The antibodies may then be used to determine the presence of such a subset of T-cells in patient's blood, using FACS or other techniques, for inhibiting the activity of such a T-cell subset, or the like. The antibodies may be introduced by conventional ways in a physiologically acceptable medium, e.g., PBS, generally having from about 0.165 mg of protein/ml.

The subject peptides and nucleic acid sequences may be derived from any convenient mammalian source, such as rodent, equine, bovine, lagomorpha, ovine, porcine, primate, or the like.

The following examples are offered by way of illustration and not by way of limitation.

### EXPERIMENTAL

A region located approximately 90 Kb 5' of C_{α} showing systematic rearrangements in adult dull CD1, CD4⁻, CD8⁻ cells and hybridomas was identified. These rearrangements were first detected by using pulsed-field gel electrophoresis (Schwartz and Cantor, Cell (1984) 37:67-75; Carle and Olson, Nucl. Acids Res. (1984) 12:5647-5664) because the J_{α}C_{α} locus spans at least 65 Kb (Wenot et al., Nature (1985) 316:832-836; Hayday et al., ibid (1985) 316:828-832). In order to analyze the rearrangement in more detail, a fragment designated A was used located 60 Kb 5' of C_{α} to isolate cosmid clones extending further 5'.

Fragment A is an oligonucleotide probe specific for the J region of the functional TCR α chain from the helper hybridoma 2B4. It was used to isolate a phage clone (λ 12) containing the genomic coding region of J_{α}2B4 (Berker et al., Nature (1985) 317:430-434) from a B10.A liver genomic library (Chien et al., Nature (1984) 309:322-326). The position of J_{α} 2B4 was mapped to about 65 Kb 5' to C_{α}. An EcoRI fragment (Fragment A) from λ 12 was then used to screen a cosmid library made of BALB/c-embryonic DNA (Cory et al., EMBO J. (1985) 4:675-681). Positive clones designated cos 2, and cos 3 were isolated and mapped with single and multiple restriction enzyme digestions. The most 5' end 2.85 Kb EcoR1 fragment from cos 3 was used to isolate clones designated cos 21 and cos 22. The DNA fragment used for further analysis was a 4.1 Kb EcoR1 to SacI subclone (pG4.1). The positions of Jₓ and Cₓ were determined by restriction enzyme mapping, Southern analysis and DNA sequencing.

The Southern analysis was performed as follows. 8 µg of EcoRI digested genomic DNA from adult liver (LV), day 14 fetal liver (FL), total thymocyte (THY), day 14 (D14) and day 15 (D15) fetal thymocytes were electrophoresed on a 0.8% agarose gel, blotted onto nylon membranes (Gene Screen, NEN Research Products) and probed with pG4.1 DNA which was radiolabeled by hexamer priming (Feinberg and Vogelstern, Anal. Biochem. (1983) 132:6-13). The blot was hybridized in 1M NaCl, 50 mM sodium phosphate, 50% formamide, 100 µg/ml salmon sperm DNA, 4 x Denhardts and 10% dextran sulfate at 40°C and washed in 2 x SSPE and 0.2xSSPE (20X SSPE is 3.6M NaCl, 0.2M Na phosphate pH 7.4 and 0.02 M EDTA) at 55°C. No difference was found in the pattern of hybridization between the C57BL/6, and BALB/c strains with respect to EcoR1 digests.

The analysis was extended to fetal thymocyte DNA's at various days in development in order to understand the kinetics of this rearrangement process. The resulting data indicate that rearrangements 5' of the C_{α} locus appear at least as early as day 14 fetal thymocytes. Therefore, it is rearranging at the same time or perhaps just before TCR β and γ genes (Born et al., Proc. Natl. Acad. Sci. USA (1985) 82:2925-2929). Although the rearranged bands become more intense and complex in day 18 and day 19 thymocyte DNA, they diminish in more mature T-cells. pG4.1 elicits a similar pattern in total thymocyte DNA as in dCD1 cells. However, these hybridizing bands are lower in intensity in cortisone treated thymocytes (which selectively enriches for the mature cells (Anderson and Blomgren, Cell Immunol. (1920) 1:362-371) and barely detectable in a preparation of splenic T-cells when equal amounts of DNA were used. Consistent with this observation is the fact that out of nine functional T-cells and hybridomas only one helper hybridoma (2B4) retains a single rearrangement on one chromosome, while the eight others have apparently deleted this region.

In order to determine the nature of these rearrangements, a λ-phage containing the rearranged fragment (2B4.Exp) from a 2B4 genomic library (Chin et al., supra) was isolated and compared with pG4.1. Restriction enzyme maps and sequence of the relevant subclones were obtained. This data establishes that there is a J_{α}-like element (Jₓ) in pG4.1 and that the rearrangement observed in 2B4 is the result of a VDJ joining event involving Jₓ and D elements 5' of Jₓ. Sequence analysis of this particular V region shows that it contains all the amino acids that are highly conserved in V_{α} genes and is in the same gene family as the V_{α} TA27 of Arden et al. (Nature (1985) 316:783-787) showing 83% homology at the DNA level and somewhat less (70%) amino acid homology. The Jₓ element has many of the conserved sequences of published J regions and particularly resembled J_{α} gene segments in size and possesses the C-terminal proline residue common to more than half of the known J_{α}'s. A conserved heptamer (AGCTGTG) and nonamer sequence (GGTTTTTTGG) separated by 13 nucleotides is located 5' of the unrearranged Jₓ sequence (GTAAGT). The particular VDJ rearrangement results in a frame shift between the conserved sequences of the V region element and those of the J, a common occurrence in immunoglobulin and T-cell receptor rearrangements, and consistent with the fact that the 2B4 hybridoma bears a functional α:β heterodimer.

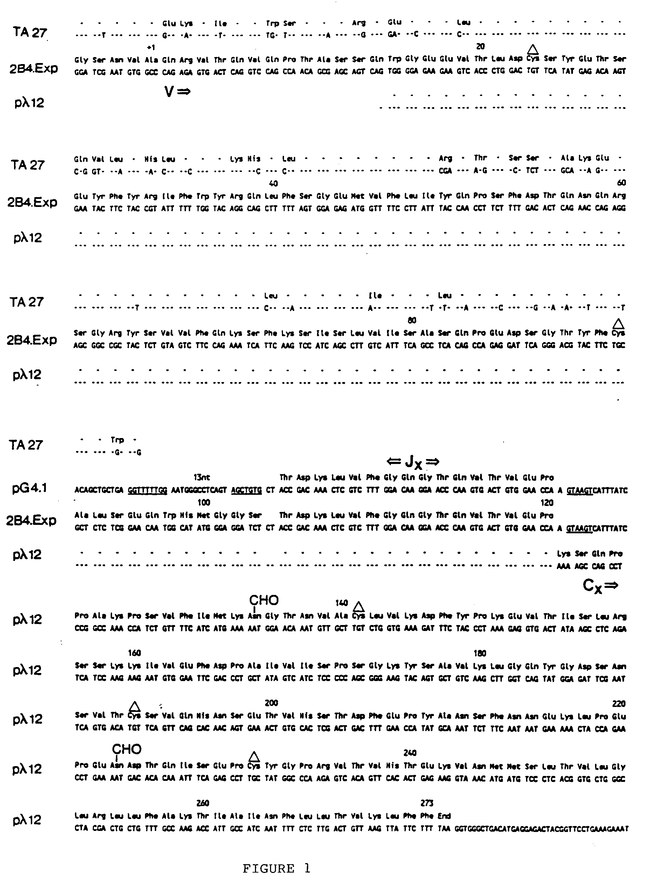

In order to determine if any transcript containing this VDJₓ sequence could be detected, the sub-clone 2B4.Exp was used as a probe for Northern analysis. Under conditions where the Jₓ element alone could hybridize, it was found to be expressed at approximately 1/20 of the level of C_{α} in 2B4 RNA, but at a higher level in new born thymocyte and adult double-negative cell RNA. A 2B4 cDNA library (-200,000 original clones) was then screened and a single clone isolated containing the VDJₓ exon described above spliced to a novel C region ("Cₓ). The Cₓ coding region exists as a single copy gene in the genome. It is located 13 kilobases 3' of the Jₓ gene segment and 70 Kb 5' of C_{α}. The genomic organization was delineated by sequence analysis. The restriction map of this cDNA clone (pλ12) and its sequence are shown in Figure 1 of the accompanying drawings.

All the DNA fragments were subcloned into the pGEM-3 vector (from Promega Biotec), and sequenced by the dideoxy chain termination method with both T7 and Sp6 promoter specific primers on double stranded plasmid DNA (Chen and Seeburg, DNA (1985) 4:165-170). All the sequences reported were done for both strands. To facilitate sequencing, deletional subclones were generated by Exo III and Mung Bean nuclease treatment (Henikoff, Gene (1984) 28:351-359). The positions of variable (V), J, constant (C) and the 3' untranslated region (3'ut) of the pλ12 cDNA clone are indicated. The shaded regions of pG4.1 and 2B4.Exp indicate sequence identity judged by DNA sequencing. The EcoRI sites at the very 5' and 3' of pλ12 are sites derived from the EcoRI linkers used for cloning. Possible N-linked glycosylation sites in the amino acid sequences are indicated by "CHO." Conserved heptamer and nonamer sequences and RNA splice sequences are underlined. Cysteine residues are indicated by a triangle. The TA27 V_{α} sequence (Arden et al., supra) is very similar to the 2B4.Exp V region and the nucleotide and amino acid homologies are indicated by dashes (-). The predicted amino acid sequence of the 2B4.Exp.pλ12 clones are numbered starting from the probable processing point at the N-terminus (Berke, supra; Arden et al., supra).

Theoretically, in an in frame message, the predicted molecular weight of this protein would be approximately 30.7 kD. The C region coding sequence shares short regions of homology with each of the previously described TCR or Ig sequences and is between 9-18% homologous at the amino acid sequence level to any one T-cell receptor sequence. It preserves many of the characteristics of T-cell receptor genes with an extra cysteine past the first domain, presumably for heterodimer formation, and also including a conserved lysine residue in the transmembrane region, a feature of all of the other T-cell receptor constant regions. It has been suggested that a lysine at this position may be important for interactions with CD3 polypeptides (Chien et al., Nature (1984) 312:31-35). This interaction has been postulated as all three of the CD3 peptides characterized to date have a conserved aspartic acid or glutamic acid residue in their apparent transmembrane regions.

The new T-cell receptor constant region sequence is only weakly homologous (9-18%) to immunoglobulin and other T-cell receptor sequences. This weak homology is shared by the previously reported C_{α} sequence and is contrasted by the C_{γ} and C_{β} constant region sequences which are much more closely related to immunoglobulins (up to 40% homology in the first domain). The possibility therefore exists that neither Cₓ nor C_{α} form any immunoglobulin-like fold and that some other structure or structures is required for part of the T-cell receptor molecule.

An intriguing similarity between Cₓ and C_{α} is that both have relatively short (14-15 amino acids) hydrophobic C-termini (counting from the conserved lysine) versus the much longer corresponding regions of C_{γ} and C_{β} (23 and 25 amino acids, respectively) which each have three charged residues near the end of the molecule (C_{β} ends in "KKKNS" and C_{γ} ends in "NEKKS"). These differences in polypeptide sequence relationships may be important in heterodimer formation and interaction with the CD3 polypeptides. Another feature of the Cₓ sequence is the similarity in the way Cₓ conserved sequences are spaced with those of C_{α}, and the relative disparity with the C_{γ} and C_{β} organization. The four T-cell receptor constant regions can be aligned by the conserved cysteine (C), lysine (K), and tryptophan (W) residues. In most cases the distances between the residues are very similar between Cₓ and C_{α} and they are very similar between γ and β. The sole exception to this observation is the distance between the second and third cysteines of C_{α} which is unusually short. Also interesting is the fact that γ and β both have a tryptophan 14 amino acids past the first conserved cysteine residue, a feature of all immunoglobulin constant regions, whereas neither Cₓ or C_{α} has a tryptophan at that position or anywhere nearby. Since it is known that α and β pair with each other, Cₓ and C_{γ} appear to be an equivalent match. A corollary is that α might be able to pair with γ, or the "x" chain pair with β. If this last possibility is true, it might help to explain the apparent mutual exclusion rules under which these two gene loci appear to operate.

In order to assess the level and distribution of x chain expression, a "Northern" blot was prepared with RNA's from immature and adult thymocyte populations and probed with Cₓ, C_{γ} and C_{α} cDNA probes. Both Cₓ and C_{γ} containing sequences are expressed at a high level in adult double-negative cell RNA and in day 15 fetal thymocyte cultures (cultured with IL-4 and tetradecylphorbol acetare although somewhat reduced in new born thymocytes, and only barely detectable in unfractionated adult thymus RNA after a long autoradiogram exposure. The level of expression in total thymus is estimated to be no more than one-tenth of that in adult double-negative cells. In contrast, C_{α} mRNA is not evident in the cultured fetal thymocytes, is just barely detectable in double-negative cell RNA, and is clearly present in adult thymocyte RNA. This data is consistent with the published results as the double-negative cell preparation was not selected with respect to the CD1 marker.

There are two predominant size classes of Cₓ specific RNA in the cell populations; 2 Kb, and 1.55 Kb (determined by using 5.1 Kb and 2.0 Kb for ribosomal 28S and 18S as markers). Using a V-region probe, hybridization is detected only with the 2.0 Kb band. D elements were identified located 5' of Jₓ and DJₓ rearrangements observed in some fetal thymocyte and adult double-negative cell hybridomas. In a double-negative hybridoma which has only one chromosome containing a DJ rearrangement, only the 1.55 Kb RNA is present. This supports that at least some of the 1.55 Kb species are DJ transcripts. This is similar to what is observed for the Ig heavy chain gene C_{µ}, and for TCR β-chain genes, where messenger RNA corresponding to rearrangements involved both VDJ and DJ are transcribed as distinct size classes.

One observation is that the size of the transcript in the 2B4 cell is 1.85 Kb. This result is reproducible and consistent with the size of the cDNA clone isolated. One explanation is that this is due to a strain polymorphism since the 2B4 transcript derives from a B10.A strain chromosome (the fusion partner, BW5147, had deleted this region from both chromosomes) and the cell populations analyzed here are from either BALB/c or BALB/c x 129 F₁'s (obtained from 3 week old BALB/c x 129F₁ thymus by testing with anti-CD4 and GK1.5 monoclonal antibody (available from Dr. A. Zlotnick)). Another explanation is differential splicing.

The particular gene described does not represent a functional rearrangement, but the structural elements of the gene are homologous to highly conserved T-cell receptor and immunoglobulin sequences. The C region sequence of the 2B4 cDNA clone was confirmed with genomic sequence analysis. Since Cₓ is a single copy gene and the intron-exon organization is very similar to those of Ig and TCR constant regions, it is unlikely to be a psuedo-gene. Furthermore, almost all γ chain cDNA clones from functional helper or cytotoxic T-cell lines represent transcripts of out-of-frame joining events and yet functional messages and proteins are detectable in other cell types. Since the cDNA isolated is from the T-helper hybridoma 2B4, which bears a functional α:β heterodimer, it is reasonable to assume that while a non-functional message is made in these cells, proteins bearing the Cₓ sequence will be identified in other T-cell types.

A functional version of the described sequence would encode a 30.7 kD polypeptide with two N-linked glycosylated sites. A fully glycosylated form would be approximately 37 kD. That is considerably smaller than the reported mouse δ chain protein which is 45 kD in its glycosylated form and becomes 37 kD after Endo F treatment (Pardolly, supra). It has recently been observed that human C_{γ} genes can be differentially spliced to include from one to three hinge-like exons which cause considerable changes in the apparent molecular weight (Littman et al., Nature (1987) 326:85-88). The 2B4 message is about 150 nucleotides shorter than the larger of the two major species containing Cₓ sequences. It therefore seems possible that similar phenomena could be occurring with the gene reported here and that the major high molecular weight species that is seen in thymocytes has an additional exon or exons compared with the 2B4 cDNA sequence. An additional 150 nucleotides of coding region sequence would give an unglycosylated molecular weight of 36.2 kD, nearly identical to that observed for the mouse δ chain.

It is evident from the above results, that the novel nucleic acid sequences may be used for identifying immature T-cells, at a particular stage in differentiation. In addition, these sequences may be used in DNA constructs to provide for expression of the subject peptide by itself, or in combination with other T-cell specific peptides, particularly other peptides involved in T-cell receptors, more particularly the β and γ peptides. The subject peptides by themselves or in combination with other peptides may be used in the binding of ligands, in a variety of environments, such as diagnostic immunoassays, affinity chromatography, and the like. In addition, the subject peptides may be used for the production of monoclonal or polyclonal antibodies to bind two T-cells carrying cross-reactive peptides, so as to activate or remove such specific T-cells.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced.

## Claims

1. A DNA construct comprising an expression cassette comprising a transcriptional and translational initiation region, a gene or fragment thereof encoding a peptide under the initiation regulatory control of said initiation region, and a translational and transcriptional termination region, said expression cassette comprising less than about five kbp of the wild-type sequence of said gene, said gene characterized by:
(a) being rearranged from germline DNA or the cDNA corresponding to the processed rearranged germline DNA;
(b) comprising V, D, J, and C regions comparable to the regions of the alpha subunit of the T-cell receptor;
(c) at a locus other than the locus of the alpha subunit of the T-cell receptor; and
(d) is expressed early in the development of the vertebrate host comprising said gene; and
wherein said fragment encodes at least eight amino acids of the C region and an oligopeptide which is immunologically cross-reactive with said peptide; joined to a heterologous DNA comprising at least one of a marker for selection, a replication system stable in a cellular host, or a synthetic linker or polylinker.

2. A DNA construct according to claim 1, wherein said transcriptional and translational initiation region is a region functional in a mammalian cell.

3. A DNA construct according to claim 1 or claim 2, wherein said gene is cDNA.

4. A transformed cell comprising a DNA construct according to any one of claims 1-3, wherein said expression cassette is joined to a marker.

5. A transformed cell according to claim 4, wherein said cell is a mammalian cell.

6. A DNA sequence of less than 5kbp comprising an open reading frame of at least twelve nucleotides of a C region of a gene and up to the sequence of said gene characterized by:
(a) being rearranged from germline DNA or the cDNA corresponding to the processed rearranged germline DNA:
(b) comprising V, D, J, and C regions comparable to the regions of the alpha subunit of the T-cell receptor;
(c) at a locus other than the locus of the alpha subunit of the T-cell receptor; and
(d) is expressed early in the development of the vertebrate host comprising said gene; and
wherein said fragment encodes at least an oligopeptide which is immunologically cross-reactive with said peptide.

7. A cDNA sequence comprising a gene characterized by:
(a) comprising V, D, J, and C regions comparable to the regions of the alpha subunit of the T-cell receptor;
(b) at a locus other than the locus of the alpha subunit of the T-cell receptor; and
(c) is expressed early in the development of the vertebrate host comprising said gene.

8. An expression cassette comprising a gene characterized by:
(a) being rearranged from germline DNA or the cDNA corresponding to the processed rearranged germline DNA;
(b) comprising V, D, J, and C regions comparable to the regions of the alpha subunit of the T-cell receptor;
(c) at a locus other than the locus of the alpha subunit of the T-cell receptor; and
(d) is expressed early in the development of the vertebrate host comprising said gene; and
wherein said fragment encodes at least an oligopeptide which is immunologically cross-reactive with said peptide, joined to at its 5' terminus to a heterologous transcriptional and translational initiation region and at its 3' terminus to a translational and transcriptional termination region.

9. A method for producing a peptide expressed by a gene or fragment thereof, said gene characterized by:
(a) being rearranged from germline DNA or the cDNA corresponding to the processed rearranged germline DNA;
(b) comprising V, D, J, and C regions comparable to the regions of the alpha subunit of the T-cell receptor;
(c) at a locus other than the locus of the alpha subunit of the T-cell receptor; and
(d) is expressed early in the development of the vertebrate host comprising said gene; and
wherein said fragment encodes at least an oligopeptide which is immunologically cross-reactive with said peptide,
by growing a cell transformed with an expression cassette comprising said gene joined at its 5' terminus to a transcriptional and translational initiation region and at its 3' terminus to a translational and transcriptional termination region.

10. A method according to claim 9, wherein said gene comprises the entire C region.
